# EUROPEAN PATENT SPECIFICATION

(11) **EP 0 623 024 B1**
(45) Date of publication and mention of the grant of the patent: **02.05.1997**
(21) Application number: 93903521.8
(22) Date of filing: 15.01.1993
(51) Int. Cl.: A61K 38/16

(54) **PHARMACEUTICAL BACTERIOCIN COMPOSITIONS AND THEIR USE FOR TREATING GASTROINTESTINAL DISORDERS**
PHARMAZEUTISCHE ZUBEREITUNGEN AUF DER BASIS VON BAKTERIOZIN UND DEREN VERWENDUNG ZUR BEHANDLUNG GASTROINTESTINALER BESCHWERDEN
COMPOSITIONS PHARMACEUTIQUES A BASE DE BACTERIOCINE SERVANT AU TRAITEMENT DES TROUBLES GASTROINTESTINAUX

(30) Priority: 17.01.1992 US 822433; 09.04.1992 US 866135
(43) Date of publication of application: 09.11.1994
(73) Proprietor: APPLIED MICROBIOLOGY, INC., Tarrytown, New York 10591 (US)
(72) Inventor: BLACKBURN, Peter, New York, NY 10036 (US); PROJAN, Steven, J., New York, NY 10021 (US); GOLDBERG, Edward, B., Newton, MA 02161 (US)
(74) Representative: Lucas, Brian Ronald
(86) International application number: US9300377
(87) International publication number: WO9313793

(56) References cited:
- WO-A-89/12399
- WO-A-92/18143
- APPL. ENVIRON. MICROBIOL., vol. 57, no. 12, 1991, pages 5613-5615; STEVENS, KELLY A. ET AL.: 'Nisin treatment for inactivtion of salmonella species and other gram-negative bacteria'

## Description

Various gastrointestinal diseases or symptoms including diarrhea, gastritis, peptic and duodenal ulcer, and gastric carcinoma are due to the presence of pathogenic microorganisms in the gastrointestinal tract. Escherichia and Salmonella, in particular, but also certain species of Clostridia, Bacillus, Bacteroides, Campylobacter and Yersinia can be responsible for diarrhea especially in neonatal farm animals. (R.E. Holland, 1990, Clin. Microbiol. Rev. 3:345, "Some infectious causes of diarrhea in young farm animals.") Helicobacter pylori are implicated in gastritis, duodenal and peptic ulcer disease. (Peterson, W.L., 1991, New Eng. J. Med. 324: 1043, "Helicobacter pylori and peptic ulcer disease") and are also associated with gastric carcinoma. (Henderson, B.E., Ross, R.K., and Pike, M.C., 1991, Science 254:1131, "Toward the primary prevention of cancer," Nomura, A. Stemmermann, G.A., Chyou, P.H., Kato,I., Perez-Perez, G. and Blaser, M.J. (1991) New Eng. J. Med. 325: 1132 "Helicobacter pylori infection and gastric carcinoma among Japanese Americans in Hawaii."; Parsonnet, J., Friedman, G.D., Vandersteen, D.P., Chang, Y., Vogelman, J.H., Orentreich,N, and Sibley, R.K. (1991) New Eng. J. Med. 325:1127; Forman, D., Sitas, F., Newell, D.G., Stacey, A.R., Boreham, J., Peto, R., Campbell, T.C., Li, J. and Chen, J. (1990) Int. J. Cancer 46:608 "Geographic association of Helicobacter pylori antibody prevalence and gastric cancer mortality in rural China").

Many gastrointestinal pathogens are gram negative bacteria. For example, Helicobacter pylori (which has also been identified in the prior art as Campylobacter pylori) is a gram negative microaerophilic bacillus that colonizes the gastric mucosa. Since 1983, when first reported in association with histologic gastritis, a relationship between suppression of H. pylori infection and improvement of gastric disorders has been noted. However, although numerous antibiotics have been tried against H. pylori infection, none have so far proved acceptable and no agent or regimen has been approved for use against this organism. Long term eradication of the organism has seldom been achieved and antibiotics themselves can produce unacceptable side effects. (Peterson, W.L., 1991, New Eng. J. Med. 324:1043 "Helicobacter pylori and peptic ulcer disease; Warren, J.R., 1983, Lancet 1:1273, "Unidentified curved bacilli on gastric epithelium in active chronic gastritis"; Morgan et al., 1988, Gastroenterology 95:1178, "Nitrofurans in the treatment of gastritis associated with Campylobacter pylori"; Glupczynski, Y. et al., 1988, Am. J. Gastroenterol. 83:365 "Campylobacter pylori-associated gastritis: a double-blind placebo controlled trial with amoxycillin"; Rauws, E.A. et al., 1988, Gastroenterology 94:33, "Campylobacter pylori-associated chronic antral active gastritis"; Glupczynski, Y. 1990 in Helicobacter pylori, gastritis, and peptic ulcer"; Malfertheiner, P., Ditschuneit, H., Eds. Springer-Verlag, Berlin, Germany pp 49-58; Rauws, E.A. and Tytgat, G.N. 1990 Lancet 335:1233 "Cure of duodenal ulcer associated with eradication of Helicobacter pylori. O'Riordan, T. et al., 1990, Gut 31:999 "Adjuvant antibiotic therapy in duodenal ulcers treated with colloidal bismuth subcitrate"; Weil, J. et al., 1990, Aliment. Pharmacol. Ther., 4:651 "Helicobacter pylori infection treated with a tripotassium dicitrato bismuthate and metronidazole combination"; Coghlan, J.G., Gilligan, D., Humphries, H., et al., 1987, Lancet 2:1109 "Campylobacter pylori and recurrence of duodenal ulcer - a 12-month follow-up study"; Marshall, B.J. Goodwin, C.S., Warren, J.R. et al., 1988, Lancet 2:1437, "Prospective double-blind trial of duodenal ulcer relapse after eradication of Campylobacter pylori").

The antimicrobial activity of the lanthionine-containing bacteriocin nisin was at one time considered restricted towards Gram positive organisms. See e.g. Hurst, A., 1981, "Nisin", Adv. in App. Micr. V. 27, pages 85-121.

Our prior PCT Application WO 89/12399 describes the activity of lanthionine-containing bacteriocins such as nisin, in the presence of a chelating agent such as EDTA or citrate, against Gram negative bacteria such as *Salmonella typhimurium*, *Escherichia coli*,*Pseudomonas aeruginosa*, *Bacteroides gingivalis*,*Actinobacillus actinomycetescomitans* and *Klebsiella pneumoniae*. The compositions of bacteriocin and chelating agent have a pH of from 5 to 8 are disclosed as useful in several connections. These include the control of bacterial contamination of food, the treatment of food packaging and handling equipment, environmental disinfection, incorporation into ointments or coatings for medicinal uses such as the treatment of infections, wound dressings or surgical implants and as a broad spectrum disinfectant for skin or oral rinses, disinfectant scrubs, wipes or lotions. Examples illustrate compositions of pH 5 to 8 as having activity against Gram negative bacteria and being useful as a food disinfectant. The application also discloses that the compositions have an enhanced activity against a broader range of Gram positive bacteria. Thus, a solution at pH 8.0 is disclosed as active against *Streptococcus mutans*, *Listeria monocytogenes* and *Coryneform bacteria* and it is suggested that a composition of pH 7.3 could be a component of a mouthwash, rinse toothpaste or other similar dentrifice.

Our prior application WO 92/18143 published 29th October 1992, designating all European countries except Ireland and Portugal, entered into the European phase as European Application 92 908 524.9. This application describes the use of a lanthionine-containing bacteriocin such as nisin for the treatment of gastric disorders associated with *Helicobacter pylori*. The bacteriocin may be combined with a chelating agent such as EDTA or citrate.

The present invention provides firstly the use of a lanthionine-containing bacteriocin, especially nisin for the manufacture of a medicament for the prevention or treatment of gastrointestinal disorders caused by pathogenic microorganisms, other than *Helicobacter pylori*, in the gastrointestinal tract. (This disclaimer does not apply to PT).

Preferably the bacteriocin is administered as a composition containing also a chelating agent such as EDTA or citrate. These compositions are stable and active at acidic pH and are useful for their antibacterial activity against Gram negative bacteria in low pH environments such as encountered in the gastrointestinal tract.

The invention further provides a pharmaceutical composition *per se*, in the form of a solid, semi-solid or capsule, suitable for administration to prevent or treat gastrointestinal disorders caused by pathogenic microorganisms in the gastrointestinal tract, said composition comprising a lanthionine-containing bacteriocin, a chelator and, as a further component different from the chelator, an acidic vehicle.

Also included in the invention is a pharmaceutical composition, in liquid form and of pH at least 2.5 but below 5.0, for use in prevention or treatment of gastrointestinal disorders caused by pathogenic microorganisms in the gastrointestinal tract, said composition comprising a lanthionine-containing bacteriocin, a chelator and, as a further component different from the chelator, an acidic vehicle.

Pharmaceutical nisin compositions according to the invention act quickly, so that when delivered into the stomach and gastrointestinal tract their activity should not be limited by the clearance rate of the stomach contents. Furthermore, unlike antibiotics, nisin compositions can be safely ingested. The pharmaceutical compositions may be used alone in treatment regimens or in combination with other pharmaceutical agents or drugs.

The above-mentioned compositions used in the invention are active in acidic pH below 5.0 and display considerable activity against Gram negative bacteria. This antibacterial activity may be useful in containing the growth of infections caused by gastrointestinal pathogens such as species of *Helicobacter*, *Escherichia*, *Salmonella*, *Bacillus*, *Clostridia*, *Bacteroides*, *Campylobacter* and *Yersinia*. Such low pH-active compositions would therefore be useful in the treatment of various diseases or symptoms due to the presence of such pathogenic bacteria.

The efficacy of the present invention has been demonstrated on E. coli bacteria which are found in the mammalian gut and are frequently responsible for gastrointestinal disorders. The survival of E. coli is unaffected by exposure to EDTA or citrate by themselves or by exposure to nisin by itself. In addition, suspensions of E. coli exposed to acid survive well in an acidic environment until the pH drops below pH 2.5. However, as is set forth below, when nisin is combined with EDTA at a range of acidic pH values, significant reduction in the viability of the bacteria was seen after only 1 minute of exposure to the nisin compositions. At pH 3.5, a reduction by more than 6 logarithms in the viable count of bacteria can be attributed to nisin after only 1 minute exposure to the nisin-chelator composition. Below pH 3.5 some reduction of the potency of the nisin compositions is apparent but, nevertheless, an approximately 1000-fold enhancement of nisin activity remains even at pH 2.5 (Table 1).

EDTA-activated nisin is bactericidal towards E. coli in the presence of various acid vehicles including acetate, citrate, lactate, and succinate, as shown in Tables 2-5. As illustrated by results obtained at pH 3.5, the rapid bactericidal activity of the nisin compositions can be influenced by the choice of acid vehicle. In all the illustrated cases, as the concentration of each acid anion is increased, the bactericidal activity of the nisin compositions is observed to decrease. Nevertheless, each of these acid vehicles is suitable for the expression of chelator-enhanced nisin activity. Exposure of the bacteria to these nisin compositions for a longer period than 1 minute is effective in reducing the number of bacteria even when the formulations contain the less effective concentrations of the acid vehicles.

### Evaluation of Germicidal Activity of Chelator-Enhanced Nisin in Acid Vehicles towards Gram Negative Bacteria.

The rapid activities of various chelator-enhanced nisin formulations were evaluated in acid vehicles in a germicidal suspension assay.

E. coli cells from an overnight Trypticase soy nutrient agar (TSA) were resuspended to a density measured as an absorbance of 1.0 at 600 nm in sterile ddH₂O. The reaction of the cells with each of the bactericidal test formulations analyzed was started by addition of 30 µl of cells to 970 µl of test formulation. The reaction mixture was incubated at 37°C for at least 1 minute and then centrifuged in a microfuge for 1 minute. The cell pellet was washed by resuspension in 1 ml of neutralization buffer. (The neutralization buffer: 50 mM Tris-HCl, pH 7.0, 5 mM MgSO₄, 20 mM CaC1₂, 0.1 M NaCl and 0.1% gelatin was prepared by first making Tris buffer and adjusting the pH. The salts and gelatin were then added and the solution stirred with heat until the solution was clear. The solution was then autoclaved for 20 min. The neutralization buffer was used without dilution.) The cells were centrifuged in a microfuge for 1 minute and resuspended in 1 ml of neutralization buffer. The viable count was determined by spreading 100 µl of bacterial suspension and serial dilutions thereof in neutralization buffer on nutrient agar and scoring surviving colonies after 24 h at 37°C. Percent survival relative to untreated controls was calculated from the scored values.

EDTA-enhanced activity of nisin is expressed at low pH. Below pH 3.5 the degree of enhancement is reduced, presumably as the carboxyl groups of the chelator are titrated. Nevertheless, an approximately 1000-fold enhancement of nisin by EDTA was observed even at pH 2.5. See results presented in Table 1. In Table 1 and subsequent Tables 2, 4 and 8-13 values preceded by "<" represent the percent survival figures, based on the initial concentration of viable bacteria (expressed as colony forming units (cfu) /ml) in the assayed bacterial suspension and the aliquot volume of said suspension taken for assay, corresponding to the detection of no surviving colonies.

**Table 1**

| **EDTA activation of Nisin towards Escherichia coli** Dependence with respect to acidic pH | | | | | | | |
|---|---|---|---|---|---|---|---|
| EDTA mM | Nisin µg/ml | pH value | | | | | |
| | | 7.0 | 2.0 | 2.5 | 3.0 | 3.5 | 4.0 |
| | | % survival at 1 min^{a} | | | | | |
| 0 | 0 | 100^{b} | - | - | - | 100 | - |
| 0 | 100 | - | 0.28 | 3.37 | 4.43 | 100 | 100 |
| 1.0 | 0 | - | 0.07 | - | 5.41 | - | - |
| 1.0 | 100 | - | 0.01 | 0.005 | 0.0007 | <10⁻⁴ | <10⁻⁴ |

| | | | | | | | |
|---|---|---|---|---|---|---|---|
| a Incubations performed at 37°C in 20 mM Na acetate buffer adjusted to pH | | | | | | | |
| b Initial viable count 4 x 10⁷ colony forming units /ml | | | | | | | |

**Table 2**

| **Chelator Activation of Nisin-Acetate towards Escherichia coli** Dependence with respect to Acetate concentration | | | | | | |
|---|---|---|---|---|---|---|
| EDTA mM | Nisin µg/ml | (% w/v) Acetate pH 3.5 | | | | |
| | | 0 | 0.1 | 0.3 | 1.0 | 3.0 |
| | | % survival at 1 min^{a} | | | | |
| 0 | 0 | 100^{b} | 55 | 80 | 90 | 10.1 |
| 0 | 100 | - | 9.6 | 70 | 100 | 100 |
| 1.0 | 0 | - | 40 | 25 | 25 | 10.7 |
| 1.0 | 100 | - | 0.0005 | <10⁻⁴ | 0.02 | 0.4 |

| | | | | | | |
|---|---|---|---|---|---|---|
| a Incubations performed at 37°C | | | | | | |
| b Initial viable count 2 x 10⁷ colony forming units /ml | | | | | | |

**Table 3**

| **Chelator Activation of Nisin-Citrate towards Escherichia coli** Dependence with respect to Citrate concentration | | | | | | |
|---|---|---|---|---|---|---|
| EDTA mM | Nisin µg/ml | (% w/v) Citrate pH 3.5 | | | | |
| | | 0 | 0.1 | 0.3 | 1.0 | 3.0 |
| | | % survival at 1 min^{a} | | | | |
| 0 | 0 | 100^{b} | 100 | 56.3 | 72.9 | 100 |
| 0 | 100 | - | 0.003 | 0.0007 | 0.56 | 14.6 |
| 1.0 | 0 | - | 100 | 50.0 | 47.9 | 100 |
| 1.0 | 100 | - | 0.0006 | 0.0002 | 0.69 | 20.8 |

| | | | | | | |
|---|---|---|---|---|---|---|
| a Incubations performed at 37°C | | | | | | |
| b Initial viable count 5 x 10⁷ colony forming units /ml | | | | | | |

**Table 4**

| **Chelator Activation of Nisin-Lactate towards Escherichia coli** Dependence with respect to Lactate concentration | | | | | | |
|---|---|---|---|---|---|---|
| EDTA mM | Nisin µg/ml | (% w/v) Lactate pH 3.5 | | | | |
| | | 0 | 0.1 | 0.3 | 1.0 | 3.0 |
| | | % survival at 1 min^{a} | | | | |
| 0 | 0 | 100^{b} | 22.2 | 46.7 | 35.6 | 4.2 |
| 0 | 100 | - | 4.4 | 0.24 | 17.8 | 26.7 |
| 1.0 | 0 | - | 5.1 | 0.46 | 0.91 | 1.58 |
| 1.0 | 100 | - | <10⁻⁴ | 0.02 | 0.20 | 2.84 |

| | | | | | | |
|---|---|---|---|---|---|---|
| a Incubations performed at 37°C | | | | | | |
| b Initial viable count 5 x 10⁷ colony forming units /ml | | | | | | |

**Table 5**

| **Chelator Activation of Nisin-Succinate towards Escherichia coli** Dependence with respect to Succinate concentration | | | | | | |
|---|---|---|---|---|---|---|
| EDTA mM | Nisin µg/ml | (% w/v) Succinate pH 3.5 | | | | |
| | | 0 | 0.1 | 0.3 | 1.0 | 3.0 |
| | | % survival at 1 min^{a} | | | | |
| 0 | 0 | 100^{b} | 85.5 | 29.9 | 36.7 | 13.9 |
| 0 | 100 | - | 18.3 | 66.6 | 83.4 | 28.4 |
| 1.0 | 0 | - | 56.8 | 63.6 | 46.2 | 43.2 |
| 1.0 | 100 | - | 0.02 | 6.21 | 14.2 | 4.73 |

| | | | | | | |
|---|---|---|---|---|---|---|
| a Incubations performed at 37°C | | | | | | |
| b Initial viable count 3.4 x 10⁶ colony forming units /ml | | | | | | |

At pH 3.5 EDTA-enhanced nisin activity was observed in the presence of all acid anions tested (see Tables 2-5). At pH 3.5, lactate at 0.3% was somewhat inhibitory to EDTA-enhanced nisin activity. Nevertheless the activity is still enhanced more than 1000-fold over 0.3% lactate alone and 0.1% lactate is not inhibitory to EDTA-enhanced nisin.

At pH 3.5, up to 0.3% acetate and 0.3% citrate appear compatible with chelator-enhanced nisin germicidal activity towards E. coli suspensions. These anions appear to show the most promise as acid vehicles to be formulated with EDTA-enhanced nisin. Citrate is a most suitable acid vehicle for EDTA-enhanced nisin compositions. Citrate is a naturally occurring food substance and intermediary metabolite and an effective enhancer of nisin bactericidal activity in its own right (Table 3). Nisin-citrate compositions can be expected to be safe and effective for containing or eliminating the growth of undesirable microorganisms in the gastrointestinal tract of humans and animals.

Citrate is a metabolite, it does not inhibit the growth of bacteria and bacteria grow well on nutrient agar supplemented with citrate. However, nisin in the presence of citrate is active against gram negative bacteria. Thus, it is possible to demonstrate the activity of nisin towards gram negative bacteria by performing growth inhibition assays on nutrient agar supplemented with various concentrations of citrate. This nisin-citrate agar assay has much more general applicability. The assay provides a method for screening potential agents other than citrate in combination with nisin for their potential properties as enhancers of nisin's inherent bactericidal activity. Examples of other organic acids in combination with nisin would include acetate, propionate, lactate, succinate, fumarate, malonate, adipate, sorbate, phosphate and ascorbate.

Other agents that potentiate nisin activity include nonionic and amphoteric surfactants and emulsifiers, quaternary compounds, monoglycerides, and fatty acids.

The nisin-citrate agar assay is performed as follows. E. coli is resuspended to an optical density of 1.0 at A₆₀₀. A 100 µl sample of the bacterial suspension is spread uniformly on Trypticase Soy nutrient agar (TSA) supplemented with various concentrations of citrate (eg. 0.1%, 0.3%, 1.0%, 3.0%) and incubated for 1 hour at 37°C. A nisin stock solution and serial dilutions thereof in 0.1% bovine serum albumin (BSA), are prepared and 5 µl are taken from each and deposited onto the growing bacterial lawn. The TSA plates are then incubated for 24 hours at 37°C. After 24 hours at 37°C, E. coli grown on TSA supplemented with citrate form a confluent lawn. The activity of nisin towards bacteria grown in the presence of citrate is demonstrated by clear zones in the bacterial lawn where the serially diluted nisin samples were deposited. The effectiveness of nisin against the gram negative bacteria can be assessed from determining the minimum amount of nisin required to produce a clear zone of growth inhibition. As the concentration of citrate in the nutrient agar is increased, less nisin is required to inhibit the growth of E. coli, as is illustrated by the data shown in Table 6.

**TABLE 6**

| The activity of Nisin towards E. coli grown on Nutrient Agar in the presence of Citrate | |
|---|---|
| % Citrate | Nisin NIC¹ |
| 0% | 3,333 µg/ml |
| 0.1% | 370 µg/ml |
| 0.3% | 123 µg/ml |
| 1.0% | 13.7 µg/ml |
| 3.0% | 0.06 µg/ml |

| | |
|---|---|
| 1 Nominal inhibitory concentration of nisin minimally required to prevent growth of E. coli strain ATCC8739 grown on Trypticase Soy Agar supplemented with the various concentrations of citrate as indicated. | |

The activity of nisin enhanced with EDTA, citrate or other chelators has also been demonstrated towards several strains of Helicobacter pylori as well as related species, particularly Campylobacter jejuni, by the germicidal suspension assay. Examples are shown in Tables 7 - 13. Freshly grown H. pylori cells, grown on a nutrient agar plate (Trypticase Soy Agar, BBL 11043, supplemented with 5% defibrinated sheep blood), were harvested and subsequently grown at 37°C for 72-96 hours in a BBL Gaspak® System chamber with BBL Campy Pak™ Microaerophilic System envelopes and using a Campylobacter microaerophilic gas generator (BBL71034). The cells were then resuspended in sterile, deionized-distilled water to a cell density of 1.0 A₆₀₀ to provide a suitable stock suspension. The assay was started by addition of 50 µl of bacterial suspension to 950 µl of test solution, incubated at 37°C for 5 minutes and then centrifuged for 1 minute in a microfuge. The cell pellet was washed by resuspension in 1 ml of the sterile neutralization buffer described previously and centrifuged for 1 minute in a microfuge. The cells were then resuspended in Brucella-Albimi broth (BBL) and serially diluted in same prior to plating on nutrient agar. The viable count was determined by spreading 100 µl of bacterial suspension and dilutions thereof on the nutrient agar described above and scoring surviving colonies after 72-96 hours' incubation at 37°C in the modified atmosphere described above. Percent survival relative to untreated controls was calculated from the scored values.

The concentration dependence of the activity of nisin towards Helicobacter pylori in the presence and absence of 0.1% citrate at pH 5.0 is illustrated by the data presented in Table 7. Although H. pylori is a gram negative bacterium, nisin, considered active only against gram positive bacteria, surprisingly exhibits some bactericidal activity towards this organism. However, the activity of nisin towards H. pylori is significantly enhanced by the presence of citrate.

The concentration dependence of the activity of nisin towards H. pylori by citrate at pH 5.0 and pH 7.0 is illustrated by the data presented in Table 8. In general, citrate by itself has little effect on the viability of this bacterial species at pH 5.0, although at pH 7.0 the viability of the organism is somewhat reduced at higher concentrations of citrate. The effects of citrate alone are surprising since citrate is a metabolite. The enhanced activity attributable to nisin in the presence of citrate is sufficient to completely kill a 10⁵ cfu/ml suspension of H. pylori within 5 minutes at 37° C.

Data presented in Table 9 illustrate that the activity of nisin towards H. pylori at pH 5.0 and pH 7.0 is also significantly enhanced in the presence of the chelator EDTA. The chelator itself has little effect on the viability of these organisms except at higher concentrations. However, the EDTA-enhanced activity attributable to nisin is sufficient to completely kill a 10⁵ cfu/ml suspension of H. pylori within 5 minutes at 37°C.

The bactericidal activity of nisin towards H. pylori in the presence or absence of citrate or EDTA over a range of pH values is illustrated by the data presented in Table 10 and Table 11, respectively. Despite the fact that H. pylori is isolated from the stomach, the lumen of which is acidic, the viability of this organism is surprisingly poor after exposure to low pH conditions. H. pylori colonizes the stomach mucosal epithelia, a less acidic microenvironment than that of the stomach lumen. Despite the limiting viability of H. pylori at low pH in these experiments, the data indicate that nisin with citrate or EDTA can be expected to be bactericidal towards H. pylori under conditions similar to those that prevail in the stomach and its mucosal epithelium where H. pylori is able to thrive.

Campylobacter jejuni is a gram negative bacterium that colonizes the intestines of birds and mammals and has been associated with food poisoning. The bactericidal activity of nisin, in the presence and absence of citrate or EDTA, towards C. jejuni is illustrated by the data presented in Table 12 and Table 13, respectively. Nisin by itself is extremely effective towards this gram negative bacterium. At pH 5.0, higher concentrations of citrate also proved to be toxic towards this bacterium. Thus, combinations of nisin with citrate or EDTA can be expected to be effective towards C. jejuni as is illustrated by the data in Tables 12 and 13.

**Table 7**

| **Bactericidal activity of Nisin towards Helicobacter pylori** Activity with respect to nisin concentration | | | | | | |
|---|---|---|---|---|---|---|
| Strain ATCC# | Citrate pH 5.0 | Nisin (µg/ml) | | | | |
| | | 0 | 10 | 30 | 100 | 300 |
| | | % survival at 5 min^{a} | | | | |
| ATCC 43579 | 0 | 100^{b} | 1.41 | 0.39 | 0.085 | 0.0056 |
| | 0.1% | 86.2 | 0.21 | 0.008 | 0.001 | 1.89x10⁻⁵ |

| | | | | | | |
|---|---|---|---|---|---|---|
| a Incubations performed at 37°C | | | | | | |
| b Initial viable count 3.19 x 10⁷ cfu/ml | | | | | | |

**Table 8**

| **Bactericidal activity of Nisin towards Helicobacter pylori** Activity with respect to citrate at pH 5.0 and pH 7.0 | | | | | | |
|---|---|---|---|---|---|---|
| Strain ATCC# | pH | Nisin µg/ml | (% w/v) Citrate | | | |
| | | | 0 | 0.1 | 0.3 | 1.0 |
| | | | % survival at 5 min^{a} | | | |
| ATCC 43579 | 5.0 | 0 | 100^{b} | 100 | 100 | 100 |
| | | 100 | 9.62 | <0.01 | <0.01 | <0.01 |
| ATCC 43504 | 5.0 | 0 | 100^{c} | 83.6 | 42.9 | 22.1 |
| | | 0 | 100^{d} | 25.8 | 4.39 | 50.9 |
| | | 100 | n.a. | 0.033 | 0.066 | 0.14 |
| | | 100 | 0.043 | <3.1x10⁻³ | 0.92 | 2.44 |
| ATCC 43579 | 7.0 | 0 | 100^{e} | 12.1 | 0.26 | 0.02 |
| | | 100 | 0.78 | <8.1x10⁻³ | <8.1x10⁻³ | <8.1x10⁻³ |
| ATCC 43504 | 7.0 | 0 | 100^{f} | 17.7 | 8.94 | 0.82 |
| | | 100 | 2.08 | <0.01 | <0.01 | <0.01 |

| | | | | | | |
|---|---|---|---|---|---|---|
| a Incubations performed at 37°C for 5 minutes | | | | | | |
| b Initial viable count 1.04 x 10⁴ cfu/ml | | | | | | |
| c Initial viable count 1.40 x 10⁵ cfu/ml | | | | | | |
| d Initial viable count 3.26 x 10⁵ cfu/ml | | | | | | |
| e Initial viable count 1.24 x 10⁵ cfu/ml | | | | | | |
| f Initial viable count 9.62 x 10⁴ cfu/ml | | | | | | |

**Table 9**

| **Bactericidal Activity of Nisin Towards Helicobacter pylori** Activity with respect to EDTA at pH 5.0 and pH 7.0 | | | | | | |
|---|---|---|---|---|---|---|
| Strain ATCC# | pH | Nisin µg/ml | (mM) EDTA | | | |
| | | | 0 | 1.0 | 10 | 100 |
| | | | % survival at 5 min^{a} | | | |
| ATCC 43579 | 5.0 | 0 | 100^{b} | 13.63 | 8.82 | 0.43 |
| | | 100 | 4.7 | <5.0x10⁻³ | <5.0x10⁻³ | <5.0x10⁻³ |
| ATCC 43526 | 7.0 | 0 | 100^{c} | 94.1 | 21.0 | 1.62 |
| | | 100 | 5.88 | <0.03 | <0.03 | <0.03 |

| | | | | | | |
|---|---|---|---|---|---|---|
| a Incubations performed at 37°C for 5 minutes | | | | | | |
| b Initial viable count 2.04 x 10⁵ cfu/ml | | | | | | |
| c Initial viable count 3.40 x 10⁴ cfu/ml | | | | | | |

**Table 10**

| **Bactericidal Activity of Nisin Towards Helicobacter pylori** Dependence with respect to citrate in pH range 2.5 to 5.0 | | | | | | | |
|---|---|---|---|---|---|---|---|
| Strain ATCC# | Citrate % | Nisin µg/ml | pH value | | | | |
| | | | 2.5 | 3.0 | 3.5 | 4.0 | 5.0 |
| | | | % survival at 5 min^{a} | | | | |
| ATCC 43579 | 0 | 0 | - | - | - | - | 100^{b} |
| | 0 | 100 | - | - | - | - | 8.6x10⁻³ ^{e} |
| | 0 | 100 | - | - | - | - | 0.30^{c} |
| | 0.1 | 0 | <5.0x10⁻⁴ | 1.1x10⁻³ | 0.18 | 9.56 | 97.2^{b} |
| | 0.1 | 100 | <5.0x10⁻⁴ | 1.1x10⁻³ | 1.1x10⁻³ | 2.0x10⁻³ | 2.7x10⁻³ ^{b} |
| ATCC 43504 | | | | | | | |
| | 0 | 0 | - | - | - | - | 100^{d} |
| | 0 | 0 | - | - | - | - | 100^{e} |
| | 0 | 100 | - | - | - | - | 0.69^{d} |
| | 0 | 100 | - | - | - | - | 4.86^{e} |
| | 0.1 | 0 | 0.14 | <0.004 | <0.004 | 0.36 | 68.5^{d} |
| | 0.1 | 100 | <0.004 | <0.004 | <0.004 | <0.004 | 0.022^{d} |
| | 0.3 | 0 | 0.027 | <0.027 | 29.7 | 17.6 | 100^{e} |
| | 0.3 | 100 | <0.027 | <0.027 | 2.7 | 0.32 | 0.18^{e} |

| | | | | | | | |
|---|---|---|---|---|---|---|---|
| a Incubations for 5 min at 37°C in citrate adjusted to pH, or 20 mM acetate, pH 5.0. | | | | | | | |
| b Initial viable count 1.85 x 10⁶ cfu/ml | | | | | | | |
| c Average of 5 experiments | | | | | | | |
| d Initial viable count 2.7 x 10⁵ cfu/ml | | | | | | | |
| e Initial viable count 3.7 x 10⁴ cfu/ml | | | | | | | |

**Table 11**

| **Bactericidal activity of Nisin Towards Helicobacter pylori** Activity with respect to EDTA in the pH range 2.5 to 5.0 | | | | | | | |
|---|---|---|---|---|---|---|---|
| Strain ATCC# | EDTA mM | Nisin µg/ml | pH value | | | | |
| | | | 2.5 | 3.0 | 3.5 | 4.0 | 5.0 |
| | | | % survival at 5 min^{a} | | | | |
| ATCC 43504 | 0 | 0 | - | - | - | - | 100^{b} |
| | 0 | 0 | - | - | - | - | 100^{c} |
| | 0 | 100 | - | - | - | - | 0.021^{d} |
| | 0 | 100 | - | - | - | - | 7.03^{e} |
| | 1.0 | 0 | 0.023 | <0.021 | 94.2 | 11.6 | 10.0^{b} |
| | 1.0 | 0 | 0.013 | 0.001 | 0.015 | 16.5 | 100^{c} |
| | 1.0 | 100 | <0.021 | <0.021 | 0.012 | <0.021 | 0.53^{b} |
| | 1.0 | 100 | <0.001 | <0.001 | 0.005 | 0.54 | 0.32^{c} |

| | | | | | | | |
|---|---|---|---|---|---|---|---|
| a Incubations for 5 min at 37°C in citrate adjusted to pH, or 20 mM acetate, pH 5.0. | | | | | | | |
| b Initial viable count 8.6 x 10⁴ cfu/ml | | | | | | | |
| c Initial viable count 9.82 x 10⁵ cfu/ml | | | | | | | |
| d Incubated in presence of 0.1% citrate | | | | | | | |
| e Incubated in presence of 20 mM acetate | | | | | | | |

**Table 12**

| **Bactericidal Activity of Nisin Towards Campylobacter jejuni ATCC29428** Dependence with respect to citrate at pH 5.0 (strain ATCC29428) | | | | |
|---|---|---|---|---|
| Nisin µg/ml | (%w/v) Citrate pH 5.0 | | | |
| | 0 | 0.1 | 0.3 | 1.0 |
| | % survival at 5 min^{a} | | | |
| 0 | 100^{b} | 2.9 | <6.4x10⁻³ | <6.4x10⁻³ |
| 100 | <6.4x10⁻³ | <6.4x10⁻³ | <6.4x10⁻³ | <6.4x10⁻³ |

| | | | | |
|---|---|---|---|---|
| a Incubations performed at 37°C for 5 minutes | | | | |
| b Initial viable count 1.57 x 10⁵ cfu/ml | | | | |

**Table 13**

| **Bactericidal Activity of Nisin Towards Campylobacter jejuni ATCC29428** Dependence with respect to EDTA at pH 5.0 (strain ATCC29428) | | | | |
|---|---|---|---|---|
| Nisin µg/ml | mM EDTA pH 5.0 | | | |
| | 0 | 1.0 | 10 | 100 |
| | % survival at 5 min^{a} | | | |
| 0 | 100^{b} | 56.5 | 76.1 | 76.1 |
| 100 | <5.0x10⁻⁴ | <5.0x10⁻⁴ | <5.0x10⁻⁴ | <5.0x10⁻⁴ |

| | | | | |
|---|---|---|---|---|
| a Incubations performed at 37°C for 5 minutes | | | | |
| b Initial viable count 1.84 x 10⁶ cfu/ml | | | | |

The activity of nisin enhanced with EDTA, citrate or other chelators can also be demonstrated towards species of Salmonella by germicidal suspension assays. Freshly grown S. typhimurium cells are taken from a nutrient agar plate (Trypticase Soy Agar, BBL11043,) grown at 37°C for 24 hours. The cells are resuspended to a cell density of 1.0 A₆₀₀ to provide a suitable stock suspension. The assay is started by addition of 30 µl of bacterial suspension to 970 µl of test solution and incubated for at least 1 minute and then centrifuged for 1 minute in a microfuge. The cell pellet is washed by resuspension 1 ml of sterile neutralization buffer, resuspended again and then serially diluted in neutralization buffer. The viable count is determined by spreading 100 ul of bacterial suspension and dilutions thereof on nutrient agar and scoring surviving colonies after 24 hours incubation at 37°C. Percent survival relative to untreated controls is calculated from the scored values.

The low-pH-active bacteriocin compositions of the invention are preferably administered orally in the form of a pharmaceutical preparation which contains an effective amount of the lanthionine-containing bacteriocin and a pharmaceutically acceptable carrier. The carrier may also include an effective amount of a chelator and/or an acidic vehicle and/or a surfactant or emulsifier, monoglyceride, or fatty acid. The lanthionine-containing bacteriocin may be selected from the group consisting of nisin, subtilin, epidermin, Pep 5, ancovenin, gallidermin, duromycin or cinnamycin. Suitable chelating agents include, but are not limited to, EDTA, CaEDTA, CaNa₂EDTA and other alkyldiamine tetracetates as well as citrate. In certain instances the chelator and the acidic vehicle can be the same, such as when the acidic vehicle and the chelator are both citrate. Suitable acidic vehicles for use in the compositions of this invention are acetate, propionate, citrate, lactate, succinate, fumarate, malonate, adipate, sorbate, phosphate and ascorbate.

The compositions of the invention are also effective at slightly acid pH levels, (e.g., pH 5.0) and even higher pH levels, (e.g., pH 8.0), against pathogenic bacteria which may inhabit the gastrointestinal tract, such as E. coli and S. typhimurium as disclosed in issued U.S. Patent No. 5,135,910.

The pharmaceutical compositions of the invention may thus also be formulated as antacid compositions or administered in combination with an antacid wherein the administration would result for instance in a higher stomach pH environment than that existing prior to administration. The nisin chelator compositions would still be effective against the pathogenic bacteria under such conditions.

The pharmaceutically acceptable carrier may be in the form of a solid, semi-solid or liquid diluent or a capsule. In certain embodiments of the invention the acidic vehicle and the pharmaceutical carrier may be the same. Other pharmaceutically acceptable carriers may be cellulose derivatives, gelatin, lactose, starch, etc.

The pharmaceutical compositions may be in the form of solutions, colloids or emulsions, powders, tablets, capsules or gels.

The dry forms of the compositions active at low pH may be pressed into tablets which may be coated with a concentrated solution of sugar and which may contain other pharmaceutically acceptable substituents such as gum arabic, gelatin, talc, or titanium dioxide and may be also coated with various dyes. Hard gelatin capsules may be prepared which contain granules of the bacteriocin, acid vehicle and chelating agent in combination with a solid carrier such as lactose, potato starch, corn starch, cellulose derivatives or gelatin.

Liquid preparations for oral administration may be prepared in the form of syrups or suspensions comprising the peptide bacteriocin, the chelating agent, the acid vehicle, and sugar, water and glycerol or propylene glycol. If desired, such liquid preparations may contain coloring agents, flavoring agents, sweeteners such as saccharin and thickening agents such as cellulose derivatives.

Delivery of a dosage could obviously be achieved by modifications of the simple aqueous formulations by inclusion of thickeners, emulsifiers, or particulates to effect a colloidal suspension. Alternatively, osmotically balanced solutions containing a suitable dosage could be administered in volumes as little as 10 ml or as large as 4 liters. Osmotically balanced solutions such as those used as gastrointestinal lavage solutions would be suitable (Di Palma, J.A. and Brady, C.E., 1989, Am. J. Gastroenterol. 84:1008, "Colon Cleansing for Diagnostic and Surgical Procedures: Polyethylene glycol Lavage Solution"; Di Palma, J.A. and Marshal, J.B., 1990, Gastrointestinal Endoscopy 1990, 36:285, "Comparison of a new Sulfate-free Polyethylene glycol Electrolyte Lavage Solution versus a Standard Solution for Colonoscopy Cleansing"; Fordtran, J.S., et al., 1990, Gastroenterol. 98:11, "A low-Sodium Solution for Gastrointestinal Lavage"). The performance of gastrointestinal lavage solutions used to cleanse the gastrointestinal tract would be expected to be improved by inclusion of the germicidal compositions described herein. The typical daily dose of the inventive compositions may vary according to the pathogenic microorganism infection being treated, the site of infection, and the symptoms of the disease being treated. In general, it is expected that oral dosages would range from 0.1 mg per dose to 300 mg per dose of lanthionine-containing bacteriocin substance, and 0.1 g per dose to 30 g per dose of chelator. For example, since the volume of stomach contents varies as a function of the time lapsed after the last meal, simple aqueous formulations suitable for gastrointestinal use may be prepared as follows: For a final concentration to be achieved in the stomach at 0.1% citrate + 0.001% nisin (10 ug/ml) delivered in 10 ml and assuming approximately 100 ml in stomach:

| | | |
|---|---|---|
| Dosage 1: 1.0 mg nisin and 0.1 g citrate | Na citrate | 1.0% |
| | nisin | 0.01% |
| | saccharin | 0.005% |
| | polysorbate | 20 1.0% |
| | glycerol | 10.0% |
| | water | 87.985% |

For a final concentration to be achieved in the stomach at 3.0% citrate + 0.03% nisin (300 ug/ml) delivered in 10 ml and assuming 100 ml in stomach:

| | | |
|---|---|---|
| Dosage 2: 30 mg nisin and 3.0 g citrate | Na citrate | 30.0% |
| | nisin | 0.3% |
| | saccharin | 0.005% |
| | polysorbate | 20 1.0% |
| | glycerol | 0.0% |
| | water | 58.695% |

For a final concentration to be achieved in the stomach at 0.1% citrate + 0.001% nisin (10 ug/ml) delivered in 10 ml and assuming 1000 ml in stomach:

| | | |
|---|---|---|
| Dosage 3: 10 mg nisin and 1.0 g citrate | Na citrate | 10.0% |
| | nisin | 0.1% |
| | saccharin | 0.005% |
| | polysorbate | 20 1.0% |
| | glycerol | 10.0% |
| | water | 78.895% |

For a final concentration to be achieved in the stomach at 3.0% citrate + 0.03% nisin (300 ug/ml) delivered in 100 ml and assuming 1000 ml in stomach:

| | | |
|---|---|---|
| Dosage 4: 300 mg nisin and 30 g citrate | Na citrate | 30.0% |
| | nisin | 0.3% |
| | saccharin | 0.005% |
| | polysorbate | 20 1.0% |
| | glycerol | 10.0% |
| | water | 58.695% |

For a final concentration to be achieved in the stomach at 3.0% citrate + 0.03% nisin (300 ug/ml) delivered in 100 ml and assuming 100 ml in stomach:

| | | |
|---|---|---|
| Dosage 5: 60 mg nisin and 6.0 g citrate | Na citrate | 6.0% |
| | nisin | 0.06% |
| | saccharin | 0.005% |
| | polysorbate | 20 1.0% |
| | glycerol | 10.0% |
| | water | 82.935% |

It is also contemplated that depending on the type of pathogenic microorganism and disease being treated, the treatment regimen may comprise other drugs and pharmaceutical agents either as part of the pharmaceutical composition being administered or in treatment regimens which combine both the low-pH-active bacteriocin composition and another drug effective for treating the gastrointestinal tract. For example, in the treatment of diarrhea which may be caused by infections of a pathogenic microorganism such as one of the species of Salmonella, the bacteriocin composition active at low pH may be administered in a pharmaceutical preparation which also contains kaolin, pectin, or some other binding agent. Such symptoms may also be treated by the concurrent administration of the bacteriocin composition active at low pH and the binding agent. In addition, antacid formulations may be used in such treatment regimens and it is not expected that the antacid will affect the activity of the nisin-chelator composition.

In treating infections of the pathogenic microorganism Helicobacter pylori, the low-pH-active bacteriocin composition may be administered in connection with another pharmaceutically active substance against H. pylori such as a bismuth salt, e.g., bismuth subcitrate or bismuth subsalicylate. The inventive compositions may be administered in connection with other agents such as cimetidine, ranitidine, omeprazole, antacids, urease inhibitors or combinations thereof in order to treat some of the diseases and symptoms associated with the presence of H. pylori in the gastrointestinal tract. It is contemplated that in these therapies the active pharmaceutical agents may be administered concurrently or intermittently with the inventive pharmaceutical compositions and the mode of administration may be varied during the course of the treatment as required.

H. pylori has been isolated from dental plaque which may constitute a reservoir for recurrent infection of the stomach (Desa; H.G., Gill, H.H., Shankaran, K., Mehta, P.R., and Prabha, S.R. (1991) Dental Plaque: a permanent reservoir of Helicobacter pylori? Scand. J. Gastroenterol. 26: 1205 and Shames, B., Krajden, S., Fukasa, M., Babida, C. and Penner, J.L. (1989) Evidence for the Occurrence of the Same Strain of Campylobacter pylori in the Stomach and Dental Plaque. J. Clin. Microbiol. 27: 2849.)

It, therefore, is anticipated that bacteriocin compositions suitable for use against H. pylori in dental plaque may be used in conjunction with the bacteriocin compositions active at low pH against H. pylori in the gastrointestinal tract.

## Claims (Claims for the following Contracting State(s): AT, BE, CH, DE, DK, ES, FR, GB, GR, IE, IT, LI, LU, MC, NL, SE)

1. The use of a lanthionine-containing bacteriocin for the manufacture of a medicament for the prevention or treatment of gastrointestinal disorders caused by pathogenic microorganisms, other than Helicobacter pylori, in the gastrointestinal tract.

2. Use according to claim 1, wherein the disorders are attributable to Gram-negative bacteria.

3. Use according to claim 1 wherein the microorganisms comprise a species of Helicobacter, Salmonella, Escherichia , Clostridia, Bacillus, Bacteroides, Campylobacter or Yersinia.

4. Use according to Claim 3 wherein the microorganisms comprise Campylobacter jejuni.

5. Use according to any preceding Claim wherein a composition comprising the bacteriocin and a chelator is used for the manufacture of the medicament.

6. Use according to Claim 5 wherein the chelator is EDTA, citrate or both EDTA and citrate.

7. Use according to Claim 5 or 6 wherein the composition comprises, as a further component different from the chelator, an acidic vehicle.

8. Use according to Claim 7 wherein the acidic vehicle is acetate, propionate, citrate, lactate, succinate, fumarate, malonate, adipate, sorbate, phosphate or ascorbate.

9. Use according to any preceding claim wherein the bacteriocin is nisin.

10. A pharmaceutical composition in the form of a solid, semi-solid or capsule, suitable for administration to prevent or treat gastrointestinal disorders caused by pathogenic microorganisms in the gastrointestinal tract, said composition comprising a lanthionine-containing bacteriocin, a chelator and, as a further component different from the chelator, an acidic vehicle.

11. A pharmaceutical composition, in liquid form and of pH at least 2.5 but below 5.0, for use in prevention or treatment of gastrointestinal disorders caused by pathogenic microorganisms in the gastrointestinal tract, said composition comprising a lanthionine-containing bacteriocin, a chelator, and, as a further component different from the chelator, an acidic vehicle.

12. A composition according to claim 11 wherein the microorganisms comprise a species of Helicobacter.

13. A composition according to claim 12 wherein the microorganisms comprise Helicobacter pylori.

14. A composition according to claim 10 in capsular form, wherein the acidic vehicle imparts a pH of at least 2.5 but below 5.0.

15. A composition according to any one of claims 10 to 14, wherein the chelator is EDTA.

16. A composition according to any one of claims 10 to 15, wherein the acidic vehicle is acetate, propionate, citrate, lactate, succinate, fumarate, malonate, adipate, sorbate, phosphate or ascorbate.

17. A composition according to any one of claims 10 to 16 wherein the bacteriocin is nisin.

18. A powder, tablet or capsule suitable for oral administration to prevent or treat gastrointestinal disorders, comprising a pharmaceutically acceptable carrier for such a formulation, nisin, EDTA and citrate.

## Claims (Claims for the following Contracting State(s): PT)

1. The use of a lanthionine-containing bacteriocin for the manufacture of a medicament for the prevention or treatment of gastrointestinal disorders caused by pathogenic microorganisms in the gastrointestinal tract.

2. Use according to claim 1, wherein the disorders are attributable to Gram-negative bacteria.

3. Use according to claim 1 wherein the microorganisms comprise a species of Helicobacter, Salmonella, Escherichia , Clostridia, Bacillus, Bacteroides, Campylobacter or Yersinia.

4. Use according to Claim 3 wherein the microorganisms comprise Helicobacter pylori or Campylobacter jejuni.

5. Use according to any preceding Claim wherein a composition comprising the bacteriocin and a chelator is used for the manufacture of the medicament.

6. Use according to Claim 5 wherein the chelator is EDTA, citrate or both EDTA and citrate.

7. Use according to Claim 5 or 6 wherein the composition comprises, as a further component different from the chelator, an acidic vehicle.

8. Use according to Claim 7 wherein the acidic vehicle is acetate, propionate, citrate, lactate, succinate, fumarate, malonate, adipate, sorbate, phosphate or ascorbate.

9. Use according to any preceding claim wherein the bacteriocin is nisin.

10. A pharmaceutical composition in the form of a solid, semi-solid or capsule, suitable for administration to prevent or treat gastrointestinal disorders caused by pathogenic microorganisms in the gastrointestinal tract, said composition comprising a lanthionine-containing bacteriocin, a chelator and, as a further component different from the chelator, an acidic vehicle.

11. A pharmaceutical composition, in liquid form and of pH at least 2.5 but below 5.0, for use in prevention or treatment of gastrointestinal disorders caused by pathogenic microorganisms in the gastrointestinal tract, said composition comprising a lanthionine-containing bacteriocin, a chelator, and, as a further component different from the chelator, an acidic vehicle.

12. A composition according to claim 11 wherein the microorganisms comprise a species of Helicobacter.

13. A composition according to claim 12 wherein the microorganisms comprise Helicobacter pylori.

14. A composition according to claim 10 in capsular form, wherein the acidic vehicle imparts a pH of at least 2.5 but below 5.0.

15. A composition according to any one of claims 10 to 14, wherein the chelator is EDTA.

16. A composition according to any one of claims 10 to 15, wherein the acidic vehicle is acetate, propionate, citrate, lactate, succinate, fumarate, malonate, adipate, sorbate, phosphate or ascorbate.

17. A composition according to any one of claims 10 to 16 wherein the bacteriocin is nisin.

18. A powder, tablet or capsule suitable for oral administration to prevent or treat gastrointestinal disorders, comprising a pharmaceutically acceptable carrier for such a formulation, nisin, EDTA and citrate.

## Patentansprüche (Patentansprüche für folgende(n) Vertragsstaat(en): AT, BE, CH, DE, DK, ES, FR, GB, GR, IE, IT, LI, LU, MC, NL, SE)

1. Verwendung eines Lanthionin-enthaltenden Bacteriocins zur Herstellung eines Medikamentes zur Vorbeugung oder Behandlung von Magen-Darm-Störungen, hervorgerufen von sich von Helicobacter pylori unterscheidenden phatogenen Mikroorganismen im Magen-Darm-Trakt.

2. Verwendung gemäß Anspruch 1, wobei die Störungen gramnegativen Bakterien zugeschrieben werden können.

3. Verwendung gemäß Anspruch 1, wobei die Mikroorganismen eine der Arten von Helicobacter, Salmonella, Escherichia, Clostridia, Bacillus, Bacteroides, Campylobacter oder Yersinia umfassen.

4. Verwendung gemäß Anspruch 3, wobei die Mikroorganismen Campylobacter jejuni umfassen.

5. Verwendung gemäß einem der vorstehenden Ansprüche, wobei eine Zusammensetzung, die das Bacteriocin und einen Chelatbildner umfaßt, für die Herstellung des Medikamentes verwandt wird.

6. Verwendung gemäß Anspruch 5, wobei der Chelatbildner EDTA, Citrat oder beides, EDTA und Citrat, ist.

7. Verwendung gemäß Anspruch 5 oder 6, wobei die Zusammensetzung ein saures Vehikel als weitere, vom Chelatbildner verschiedene Komponente umfaßt.

8. Verwendung gemäß Anspruch 7, wobei das saure Vehikel Acetat, Propionat, Citrat, Lactat, Succinat, Fumarat, Malonat, Adipat, Sorbat, Phosphat oder Ascorbat ist.

9. Verwendung gemäß einem der vorstehenden Ansprüche, wobei Nisin das Bacteriocin ist.

10. Pharmazeutische Zusammensetzung in Form eines Feststoffes, Halbfeststoffes oder einer Kapsel, geeignet für die Verabreichung, um Magen-Darm-Störungen, hervorgerufen durch pathogene Mikroorganismen im Magen-Darm-Trakt, vorzubeugen oder zu behandeln, wobei die Zusammensetzungen ein Lanthionin-enthaltendes Bacteriocin, einen Chelatbildner und als weitere, vom Chelatbildner verschiedene Komponente ein acides Vehikel umfassen.

11. Pharmazeutische Zusammensetzung in flüssiger Form und von einem pH von wenigstens 2,5 aber unter 5,0 für den Gebrauch bei der Vorbeugung oder der Behandlung von Magen-Darm-Störungen, hervorgerufen durch pathogene Mikroorganismen im Magen-Darm-Trakt, wobei die Zusammensetzung ein Lanthionin-enthaltendes Bacteriocin, einen Chelatbildner und als weitere, vom Chelatbildner verschiedene Komponente ein acides Vehikel umfaßt.

12. Zusammensetzung gemäß Anspruch 11, wobei der Mikroorganismus eine Art von Helicobacter umfaßt.

13. Zusammensetzung gemäß Anspruch 12, wobei der Mikroorganismus Helicobacter pylori umfaßt.

14. Zusammensetzung gemäß Anspruch 10 in Kapselform, wobei durch das acide Vehikel ein pH von wenigstens 2,5 aber unter 5,0 gegeben wird.

15. Zusammensetzung gemäß einem der Ansprüche 10 bis 14, wobei der Chelatbildner EDTA ist.

16. Zusammensetzung gemäß einem der Ansprüche 10 bis 15, wobei das acide Vehikel Acetat, Propionat, Citrat, Lactat, Succinat, Fumarat, Malonat, Adipat, Sorbat, Phosphat oder Ascorbat ist.

17. Zusammensetzung gemäß einem der Ansprüche 10 bis 16, wobei das Bacteriocin Nisin ist.

18. Puder, Tablette oder Kapsel, welche(s) einen, für solch eine Formulierung pharmazeutisch akzeptablen Träger, Nisin, EDTA und Citrat umfaßt und welche(s) für die orale Verabreichung, um Magen-Darm-Störungen vorzubeugen oder zu behandeln, geeignet ist.

## Patentansprüche (Patentansprüche für folgende(n) Vertragsstaat(en): PT)

1. Verwendung eines Lanthionin-enthaltenden Bacteriocins zur Herstellung eines Medikamentes zur Vorbeugung oder Behandlung von Magen-Darm-Störungen, hervorgerufen durch pathogene Mikroorganismen im Magen-Darm-Trakt.

2. Verwendung gemäß Anspruch 1, wobei die Störungen gramnegativen Bakterien zugeschrieben werden können.

3. Verwendung gemäß Anspruch 1, wobei die Mikroorganismen eine der Arten von Helicobacter, Salmonella, Escherichia, Clostridia, Bacillus, Bacteroides, Campylobacter oder Yersinia umfassen.

4. Verwendung gemäß Anspruch 3, wobei die Mikroorganismen Helicobacter pylori oder Campylobacter jejuni umfassen.

5. Verwendung gemäß einem der vorstehenden Ansprüche, wobei eine Zusammensetzung, die das Bacteriocin und einen Chelatbildner umfaßt, für die Herstellung des Medikamentes verwandt wird.

6. Verwendung gemäß Anspruch 5, wobei der Chelatbildner EDTA, Citrat oder beides, EDTA und Citrat, ist.

7. Verwendung gemäß Anspruch 5 oder 6, wobei die Zusammensetzung ein saures Vehikel als weitere, vom Chelatbildner verschiedene Komponente umfaßt.

8. Verwendung gemäß Anspruch 7, wobei das saure Vehikel Acetat, Propionat, Citrat, Lactat, Succinat, Fumarat, Malonat, Adipat, Sorbat, Phosphat oder Ascorbat ist.

9. Verwendung gemäß einem der vorstehenden Ansprüche, wobei Nisin das Bacteriocin ist.

10. Pharmazeutische Zusammensetzung in Form eines Feststoffes, Halbfeststoffes oder einer Kapsel, geeignet für die Verabreichung, um Magen-Darm-Störungen, hervorgerufen durch pathogene Mikroorganismen im Magen-Darm-Trakt, vorzubeugen oder zu behandeln, wobei die Zusammensetzungen ein Lanthionin-enthaltendes Bacteriocin, einen Chelatbildner und als weitere, vom Chelatbildner verschiedene Komponente ein acides Vehikel umfassen.

11. Pharmazeutische Zusammensetzung in flüssiger Form und von einem pH von wenigstens 2,5 aber unter 5,0 für den Gebrauch bei der Vorbeugung oder der Behandlung von Magen-Darm-Störungen, hervorgerufen durch pathogene Mikroorganismen im Magen-Darm-Trakt, wobei die Zusammensetzung ein Lanthionin-enthaltendes Bacteriocin, einen Chelatbildner und als weitere, vom Chelatbildner verschiedene Komponente ein acides Vehikel umfaßt.

12. Zusammensetzung gemäß Anspruch 11, wobei der Mikroorganismus eine Art von Helicobacter umfaßt.

13. Zusammensetzung gemäß Anspruch 12, wobei der Mikroorganismus Helicobacter pylori umfaßt.

14. Zusammensetzung gemäß Anspruch 10 in Kapselform, wobei durch das acide Vehikel ein pH von wenigstens 2,5 aber unter 5,0 gegeben wird.

15. Zusammensetzung gemäß einem der Ansprüche 10 bis 14, wobei der Chelatbildner EDTA ist.

16. Zusammensetzung gemäß einem der Ansprüche 10 bis 15, wobei das acide Vehikel Acetat, Propionat, Citrat, Lactat, Succinat, Fumarat, Malonat, Adipat, Sorbat, Phosphat oder Ascorbat ist.

17. Zusammensetzung gemäß einem der Ansprüche 10 bis 16, wobei das Bacteriocin Nisin ist.

18. Puder, Tablette oder Kapsel, welche(s) einen, für solch eine Formulierung pharmazeutisch akzeptablen Träger, Nisin, EDTA und Citrat umfaßt und welche(s) für die orale Verabreichung, um Magen-Darm-Störungen vorzubeugen oder zu behandeln, geeignet ist.

## Revendications (Revendications pour l'(les) Etat(s) contractant(s) suivant(s): AT, BE, CH, DE, DK, ES, FR, GB, GR, IE, IT, LI, LU, MC, NL, SE)

1. Utilisation d'une bactériocine contenant de la lanthionine pour la fabrication d'un médicament destiné au traitement ou à la prévention de troubles gastrointestinaux provoqués dans le tractus gastrointestinal par des micro-organismes pathogènes autres que Helicobacter pylori

2. Utilisation selon la revendication 1, dans laquelle les troubles sont attribuables à des bactéries Gram négatif.

3. Utilisation selon la revendication 1, dans laquelle les micro-organismes comprennent une espèce de Helicobacter, Salmonella, Escherichia, Clostridia, Bacillus, Bacteroïdes, Campylobacter ou Yersinia.

4. Utilisation selon la revendication 3, dans laquelle les micro-organismes comprennent Campylobacter jejuni

5. Utilisation selon l'une quelconque des revendications précédentes, dans laquelle, pour la fabrication du médicament, on utilise une composition comprenant la bactériocine et un agent chélatant.

6. Utilisation selon la revendication 5, dans laquelle l'agent chélatant est l'EDTA, du citrate ou à la fois l'EDTA et du citrate.

7. Utilisation selon la revendication 5 ou 6, dans laquelle la composition comprend, en tant que constituant supplémentaire différent de l'agent chélatant, un véhicule acide.

8. Utilisation selon la revendication 7, dans laquelle le véhicule acide est de l'acétate, du propionate, du citrate, du lactate, du succinate, du fumarate, du malonate, de l'adipate, du sorbate, du phosphate ou de l'ascorbate.

9. Utilisation selon l'une quelconque des revendications précédentes, dans laquelle la bactériocine est la nisine.

10. Composition pharmaceutique se présentant sous la forme d'un solide, semi-solide ou gélule, appropriée pour l'administration destinée au traitement ou à la prévention de troubles gastrointestinaux provoqués dans le tractus gastrointestinal par des micro-organismes pathogènes, ladite composition comprenant une bactériocine contenant de la lanthionine, un agent chélatant et, en tant que constituant supplémentaire différent de l'agent chélatant, un véhicule acide.

11. Composition pharmaceutique se présentant sous forme liquide et ayant un pH d'au moins 2,5 mais inférieur à 5,0, destinée à être utilisée dans le traitement ou la prévention de troubles gastrointestinaux provoqués dans le tractus gastrointestinal par des micro-organismes pathogènes, ladite composition comprenant une bactériocine contenant de la lanthionine, un agent chélatant et, en tant que constituant supplémentaire différent de l'agent chélatant, un véhicule acide.

12. Composition selon la revendication 11, dans laquelle les micro-organismes comprennent une espèce d'Helicobacter.

13. Composition selon la revendication 12, dans laquelle les micro-organismes comprennent Helicobacter pylori

14. Composition selon la revendication 10, se présentant sous la forme de gélule, dans laquelle le véhicule acide confère un pH d'au moins 2,5 mais inférieur à 5,0.

15. Composition selon l'une quelconque des revendications 10 à 14, dans laquelle l'agent chélatant est l'EDTA.

16. Composition selon l'une quelconque des revendications 10 à 15, dans laquelle le véhicule acide est de l'acétate, du propionate, du citrate, du lactate, du succinate, du fumarate, du malonate, de l'adipate, du sorbate, du phosphate ou de l'ascorbate.

17. Composition selon l'une quelconque des revendications 10 à 16, dans laquelle la bactériocine est la nisine.

18. Poudre, comprimé ou gélule approprié pour l'administration par voie orale, destiné au traitement ou à la prévention de troubles gastrointestinaux, comprenant un véhicule pharmaceutiquement acceptable pour une formulation de ce type, de la nisine, de l'EDTA et du citrate.

## Revendications (Revendications pour l'(les) Etat(s) contractant(s) suivant(s): PT)

1. Utilisation d'une bactériocine contenant de la lanthionine pour la fabrication d'un médicament destiné au traitement ou à la prévention de troubles gastrointestinaux provoqués par des micro-organismes pathogènes dans le tractus gastrointestinal.

2. Utilisation selon la revendication 1, dans laquelle les troubles sont attribuables à des bactéries Gram négatif.

3. Utilisation selon la revendication 1, dans laquelle les micro-organismes comprennent une espèce de Helicobacter, Salmonella, Escherichia, Clostridia, Bacillus, Bacteroïdes, Campylobacter ou Yersinia.

4. Utilisation selon la revendication 3, dans laquelle les micro-organismes comprennent Helicobacter pylori ou Campylobacter jejuni

5. Utilisation selon l'une quelconque des revendications précédentes, dans laquelle, pour la fabrication du médicament, on utilise une composition comprenant la bactériocine et un agent chélatant.

6. Utilisation selon la revendication 5, dans laquelle l'agent chélatant est l'EDTA, du citrate ou à la fois l'EDTA et du citrate.

7. Utilisation selon la revendication 5 ou 6, dans laquelle la composition comprend, en tant que constituant supplémentaire différent de l'agent chélatant, un véhicule acide.

8. Utilisation selon la revendication 7, dans laquelle le véhicule acide est de l'acétate, du propionate, du citrate, du lactate, du succinate, du fumarate, du malonate, de l'adipate, du sorbate, du phosphate ou de l'ascorbate.

9. Utilisation selon l'une quelconque des revendications précédentes, dans laquelle la bactériocine est la nisine.

10. Composition pharmaceutique se présentant sous la forme d'un solide, semi-solide ou gélule, appropriée pour l'administration destinée au traitement ou à la prévention de troubles gastrointestinaux provoqués dans le tractus gastrointestinal par des micro-organismes pathogènes, ladite composition comprenant une bactériocine contenant de la lanthionine, un agent chélatant et, en tant que constituant supplémentaire différent de l'agent chélatant, un véhicule acide.

11. Composition pharmaceutique se présentant sous forme liquide et ayant un pH d'au moins 2,5 mais inférieur à 5,0, destinée à être utilisée dans le traitement ou la prévention de troubles gastrointestinaux provoqués dans le tractus gastrointestinal par des micro-organismes pathogènes, ladite composition comprenant une bactériocine contenant de la lanthionine, un agent chélatant et, en tant que constituant supplémentaire différent de l'agent chélatant, un véhicule acide.

12. Composition selon la revendication 11, dans laquelle les micro-organismes comprennent une espèce d'Helicobacter.

13. Composition selon la revendication 12, dans laquelle les micro-organismes comprennent Helicobacter pylori

14. Composition selon la revendication 10, se présentant sous la forme de gélule, dans laquelle le véhicule acide confère un pH d'au moins 2,5 mais inférieur à 5,0.

15. Composition selon l'une quelconque des revendications 10 à 14, dans laquelle l'agent chélatant est l'EDTA.

16. Composition selon l'une quelconque des revendications 10 à 15, dans laquelle le véhicule acide est de l'acétate, du propionate, du citrate, du lactate, du succinate, du fumarate, du malonate, de l'adipate, du sorbate, du phosphate ou de l'ascorbate.

17. Composition selon l'une quelconque des revendications 10 à 16, dans laquelle la bactériocine est la nisine.

18. Poudre, comprimé ou gélule approprié pour l'administration par voie orale, destiné au traitement ou à la prévention de troubles gastrointestinaux, comprenant un véhicule pharmaceutiquement acceptable pour une formulation de ce type, de la nisine, de l'EDTA et du citrate.
